# EUROPEAN PATENT APPLICATION

(11) **EP 4 112 051 A1**
(43) Date of publication of application: **04.01.2023**
(21) Application number: 21305906.6
(22) Date of filing: 30.06.2021
(51) Int. Cl.: A61K 31/385, A61K 39/00, A61K 45/06, A61P 9/00, A61P 29/00, A61P 37/00, A61P 43/00

(54) **ANETHOLE TRITHIONE FOR THE TREATMENT OF VASCULITIDES**

(71) Applicant: OP2 DRUGS, 33604 Pessac Cedex (FR)
(72) Inventor: LE GRAND, Bruno, 81220 Teyssode (FR); ROUBENNE, Lukas, 33600 Pessac (FR)
(74) Representative: Icosa

(57) **Abstract**

The present invention relates to anethole trithione (ATT) for use for preventing, treating, and/or lessening the severity or progression of, a vasculitis in a subject in need thereof.

## Description

### FIELD OF INVENTION

The present invention relates to anethole trithione (ATT) for use for preventing, treating, and/or lessening the severity or progression of, a vasculitis in a subject in need thereof.

### BACKGROUND OF INVENTION

Systemic vasculitides are a heterogeneous group of diseases characterized by damages of the blood vessel walls, which can result in their obstruction and/or aneurysm formation.

According to the International Chapel Hill Consensus Conference on the Nomenclature of Vasculitides (CHCC 2012), vasculitides can be classified according to:
a) the size of vessels involved:
   - large vessel vasculitis (*e.g*., giant cell arteritis, Takayasu arteritis, etc.),
   - medium vessel vasculitis (*e.g*., giant cell arteritis, Takayasu arteritis, polyarteritis nodosa, Kawasaki disease, etc.),
   - small vessel vasculitis (*e.g*., antineutrophil cytoplasmic antibody (ANCA)-associated vasculitis, microscopic polyangiitis, Wegener's disease, Churg-Strauss syndrome, anti-glomerular basement membrane (GBM) disease, Henoch-Schönlein purpura, cryoglobulinemic vasculitis, hypocomplementemic urticarial vasculitis, thromboangiitis obliterans, etc.),
   - variable vessel vasculitis (Behçet's disease, Cogan's disease, etc.),
b) the organs or tissues involved:
   - single organ vasculitis (*e.g*., cutaneous leukocytoclastic angiitis, cutaneous arteritis, primary central nervous system vasculitis, isolated aortitis, etc.),
c) association with systemic diseases:
   - vasculitis associated with systemic disease *(e.g.,* lupus vasculitis, rheumatoid vasculitis, sarcoid vasculitis, etc.),
   - vasculitis associated with a probable etiology (*e.g*., hepatitis C virus-associated cryoglobulinemic vasculitis, hepatitis B virus-associated vasculitis, syphilis-associated aortitis, drug-associated immune complex vasculitis, drug-associated ANCA-associated vasculitis, cancer-associated vasculitis, etc.).

Almost all primary vasculitides can target the heart; even if cardiac manifestations are rarely predominant, they can be life threatening and, therefore, require specific diagnostic and therapeutic strategies. This complication is more frequent in children (Weiss, 2012. Pediatr Clin North Am. 59(2):407-23), in adults with a systemic inflammatory state (Sy et al., 2016. Semin Arthritis Rheum. 45(4):475-82) or in patients with cardiovascular comorbidity. Cardiac complications of vasculitides, like myocarditis, coronary arteritis, coronary disease, incidence of ventricular arrhythmias and valvular disease, can lead to congestive heart failure and represent poor prognostic factors that require aggressive therapy.

Various pathogenic mechanisms have been implicated in the induction of vasculitis, including cell-mediated inflammation, immune complex-mediated inflammation and autoantibody-mediated inflammation.

The contribution of innate immune cells toward the pathogenesis of different types of vasculitides has been described in the art, in particular of macrophages, neutrophils, natural killer (NK) cells and γδ T cells.

Macrophages in particular are resident phagocytic cells, which are critically involved in host defense as well as tissue repair and healing. More recent evidence places macrophages at the center of steady-state tissue homeostasis, dealing with waste products and tissue regeneration. Macrophages are equipped with pattern recognition receptors (PRRs), which interact with pathogen-associated molecular patterns, enabling them to efficiently phagocytose pathogens and infected cells as well as secrete defense-relevant mediators and inflammatory cytokines. Macrophages have further roles as antigen-presenting cells, effectively bridging innate and adaptive immunity. In a feed-forward loop, the activation of antigen-specific T cells results in the amplification of macrophage response.

Macrophages are located in all organs to detect, ingest and process debris, dead cells and foreign materials and are numerous in chronically inflamed, nonhealing lesions, such as the atherosclerotic plaque. Macrophages are now also recognized as key players in vasculitides: in several of the vasculitic syndromes (giant cell arteritis, Takayasu arteritis, Kawasaki disease, ANCA-associated vasculitis, or primary central nervous system vasculitis [a.k.a. primary angiitis of the central nervous system], to name but a few), granuloma formation can occur, with highly activated, tissue-destructive macrophages and surrounding T cells forming complex lymphoid microstructures.

It is well known that macrophages can live in healthy tissues for extended time periods, and multiple subsets of tissue-residing macrophages have been identified, such as microglia, dermal macrophages, and splenic marginal zone and metallophilic macrophages. In contrast with such resident macrophages, vasculitic conditions can recruit circulating monocytes and develop them into macrophages.

The current paradigm holds that macrophages differentiate from monocytes once they transition from the circulation into tissue. The steps that regulate monocyte entry into the specialized tissue site of the arterial wall are independent of the source of the cells and depend on the upregulation of molecules that mediate the arrest of circulating monocytes by the leukocyte adhesion cascade on activated endothelial cells (ECs).

The pathogenic role of macrophages in vasculitides lies in their ability to secrete soluble factors, such as cytokines, chemokines, growth factors and enzymes. Secretion of these soluble factors may represent a major amplification system in vasculitides. Moreover, macrophages form granulomatous infiltrates in the vessel wall, leading to wall vascularization, loss of medial smooth muscle cells, destruction of elastic membranous lamellae and elastin fibers in the medial layer, and growth of lumen-stenosing neointima.

Kawasaki disease (KD), which is a form of vasculitis, was first described in Japan by Tomisaku Kawasaki in 1967, and is associated with cardiovascular manifestations and complications, in particular a left ventricular (LV) dysfunction occurring in 50 % to 70 % of patients during the acute stage (Yu et al., 2010. Pediatr Cardiol. 31(6):807-12; Dionne et al., 2018. Int J Rheum Dis. 21(1):45-49), representing the major contributors to morbidity and mortality.

The annual incidence of hospitalizations of U.S. patients with KD (19/100,000 children younger than five) has not changed significantly over the last 20 years (Uehara & Belay, 2012. J Epidemiol. 22(2):79-85). Asian and black Americans are 2.5- and 1.5-times more likely to develop KD than whites, respectively, suggesting a genetic link (Uehara & Belay, 2012. J Epidemiol. 22(2):79-85; Onouchi, 2012. Circ J. 76(7):1581-6). Approximately 75% to 80% of cases in the United States occur in children younger than five; the median age at diagnosis is 1.5 years, and the male-to-female ratio is approximately 1.5:1.4. Peak incidence occurs between January and March, also suggesting an environmental contribution (Uehara & Belay, 2012. J Epidemiol. 22(2):79-85; Burns et al., 2013. PLoS One. 8(9):e74529).

Although all the triggering agents of KD remain unknown, an infiltration of the coronary artery wall by immune cells has also been described (Noval Rivas & Arditi, 2020. Nat Rev Rheumatol. 16(7):391-405). In particular, immunohistochemical analysis of human post-mortem tissues showed accumulation in the arterial wall of monocytes, macrophages and neutrophils (Takahashi et al., 2018. Int J Rheum Dis. 21(1):31-35). These cells synthesize and secrete various inflammatory cytokines and chemokines including tumor necrosis factor (TNF)-α, interferon (IPN)-γ and interleukin-6 (IL-6), which activate the endothelial cells to cause vasculitic syndromes.

Intravenous immunoglobulin (IVIg) and high-dose aspirin have traditionally been the cornerstones of KD management, although the role of aspirin has been called into question (Terai et al., 1997. J Pediatr. 131(6):888-93; Lee et al., 2013. Korean Circ J. 43(3):182-6). Corticosteroids have also been evaluated for the treatment of acute and refractory KD. A meta-analysis (n = 1,011) found that using corticosteroids in addition to IVIg as initial treatment significantly reduced the risk of coronary abnormalities compared with IVIg alone (odds ratio [OR] = 0.3; 95% [CI]: 0.20-0.46) (Chen et al., 2013. Heart. 99(2):76-82). IVIg prevents the development of coronary aneurysms in a dose-dependent fashion; its mechanism of action is unknown, but effects may be from its modulation of cytokine production, influence on T cell activity, and suppression of antibody synthesis (Newburger et al., 2016. JAm Coll Cardiol. 67(14):1738-49). However, coronary artery aneurysms continue to develop in 25 % to 29% of affected children, making it the leading cause of acquired heart disease in children in the developed countries (Dionne et al., 2019. Pediatrics. 143(6): e20183341).

Because available treatment like IVIg associated with corticosteroid only weakly reduce the incidence of coronary artery aneurysms (Dionne et al., 2019. Pediatrics. 143(6): e20183341), there is thus still a great interest to identify new compounds able to reduce the genesis of vasculitis, and especially KD.

Here, the Inventors have surprisingly shown that anethol trithione (also named ATT, AOL or 5-(4-methoxyphenyl)-3*H*-1,2-dithiole-3-thione) is capable (i) of reducing the contractility of arterial vessel with endothelial dysfunction and preserving arterial vessel relaxation (in other words, preventing blood vessel vasoconstriction or promoting blood vessel vasodilation), and (ii) of inhibiting the secretion of soluble factors by macrophages, in particular of TNF-α; hence paving the way to a new treatment for preventing, treating and/or lessening the severity or progression of vasculitides, whatever the blood vessel size.

### SUMMARY

The present invention relates to anethole trithione (ATT), for use in preventing, treating, and/or lessening the severity or progression of, a vasculitis in a subject in need thereof.

In one embodiment, preventing, treating, and/or lessening the severity or progression of the vasculitis comprises reducing or inhibiting macrophage activation.

In one embodiment, reducing or inhibiting macrophage activation comprises reducing the release of tumor necrosis factor (TNF)-α by said macrophages.

In one embodiment, preventing, treating, and/or lessening the severity or progression of the vasculitis comprises reducing the contractility of arterial vessels with endothelial dysfunction and/or preventing arterial vessel vasoconstriction and/or promoting arterial vessel vasodilation.

In one embodiment, preventing, treating, and/or lessening the severity or progression of the vasculitis comprises reducing the contractility of de-endothelialized arterial vessels.

In one embodiment, the vasculitis is selected from the group consisting of Kawasaki disease, giant cell arteritis, Takayasu arteritis, polyarteritis nodosa, antineutrophil cytoplasmic antibody (ANCA)-associated vasculitis, microscopic polyangiitis, Wegener's disease, Churg-Strauss syndrome, anti-glomerular basement membrane (GBM) disease, Henoch-Schönlein purpura, cryoglobulinemic vasculitis, hypocomplementemic urticarial vasculitis, thromboangiitis obliterans, Behçet's disease, Cogan's disease, cutaneous leukocytoclastic angiitis, cutaneous arteritis, primary central nervous system vasculitis, isolated aortitis, lupus vasculitis, rheumatoid vasculitis, sarcoid vasculitis, hepatitis C virus-associated cryoglobulinemic vasculitis, hepatitis B virus-associated vasculitis, syphilis-associated aortitis, drug-associated immune complex vasculitis, drug-associated ANCA-associated vasculitis, and cancer-associated vasculitis.

In one embodiment, the vasculitis is Kawasaki disease.

In one embodiment, the vasculitis is Kawa-COVID-19.

In one embodiment, ATT is to be administered before, concomitantly with or after an additional therapeutic agent.

In one embodiment, the additional therapeutic agent is selected from the group consisting of intravenous immunoglobulin (IVIg), aspirin, corticosteroids, immunosuppressor agents and alkylating agents.

In one embodiment, the additional therapeutic agent is IVIg.

In one embodiment, the subject is a child or a teenager.

### DEFINITIONS

**"Anethole trithione"** or **"ATT"** or **"AOL"** or **"5-(4-methoxyphenyl)-3*H*-1,2-dithiole-3-thione"** all refer to a substituted dithiolthione with the following formula:

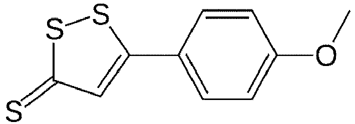

**"Lessen the severity or progression"** and any declensions thereof, with reference to a vasculitis, refers to the partial alleviation, inhibition, or mitigation of the vasculitis and/or of symptoms or complications associated therewith.

**"Pharmaceutically acceptable excipient"** refers to any inactive ingredient which is required for the formulation of an active agent in a suitable dosage form without producing an adverse, allergic or other untoward reaction when administered to an animal, preferably a human. It includes any and all any and all solvents, diluents carriers, fillers, bulking agents, binders, disintegrants, polymer, lubricant, glidant, surfactants, isotonic agents, thickening or emulsifying agents, stabilizers, absorption accelerators, flavoring agents, preservatives, antioxidants, buffering agents, or any combination thereof. For human administration, preparations should meet sterility, pyrogenicity, general safety and purity standards as required by regulatory offices, such as, *e.g.,* FDA Office or EMA.

**"Prevention"** and any declensions thereof, with reference to a vasculitis, refers to the reduction or elimination of the occurrence of the vasculitis and/or of symptoms or complications associated therewith.

**"Salt"** of the compounds of the invention is used herein to describe the acid addition and base salts thereof. Suitable acid addition salts are formed from acids which form non-toxic salts. Non-limiting examples include the acetate, trifluoroacetate, adipate, aspartate, benzoate, besylate, bicarbonate/carbonate, bisulphate/sulphate, borate, tetrafluoroborate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Non-limiting examples include the aluminium, arginine, benzathine, calcium, choline, diethylamine, diolamine, glycine, lysine, magnesium, meglumine, olamine, potassium, sodium, tromethamine, 2-(diethylamino)ethanol, ethanolamine, morpholine, 4-(2-hydroxyethyl)morpholine and zinc salts. Hemisalts of acids and bases may also be formed, for example, hemisulphate and hemicalcium salts. Preferred, pharmaceutically acceptable salts include hydrochloride/chloride, hydrobromide/bromide, bisulphate/sulphate, nitrate, citrate, and acetate.

**"Solvate"** is used herein to describe a compound in this invention that contains stoichiometric or sub-stoichiometric amounts of one or more pharmaceutically acceptable solvent molecule such as ethanol or water. The term **"hydrate"** refers to when said solvent is water.

**"Subject"** refers to an animal, including a human. In the sense of the present invention, a subject may be a **"patient",** *i.e.,* a person receiving medical attention, undergoing or having underwent a medical treatment, or monitored for the development of a disease.

**"Symptoms associated with a vasculitis"** and any declensions thereof refers to clinical signs and features giving an indication to the diagnosis of a vasculitis. These clinical signs and features vary depending on which blood vessels and, as a result, which organ systems are affected. However, general symptoms that many people with vasculitis experience include, without limitation, fever, fatigue, loss of appetite, unintentional weight loss, muscle and joint pain, and nerve problems such as numbness or weakness. Symptoms associated with some specific types of vasculitis include:
- Behcet's syndrome: mouth and genital ulcers; eye inflammation; acne-like lesions on the skin;
- Churg-Strauss syndrome: asthma;
- cryoglobulinemic vasculitis: purpura on the lower extremities; arthritis; weakness; nerve damage (neuropathy);
- giant cell arteritis: headaches; scalp tenderness; jaw pain while chewing; blurred or double vision; blindness;
- Henoch-Schönlein purpura: abdominal pain; blood in the urine; joint pain; purpura on the buttocks, legs and feet;
- Kawasaki disease: fever; skin rash; eye inflammation;
- microscopic polyangiitis: skin lesions; fever; unintentional weight loss; glomerulonephritis; nerve damage;
- polyarteritis nodosa: purpura; skin ulcers; muscle and joint pain; abdominal pain; kidney dysfunctions;
- Takayasu arteritis: feeling of numbness or cold in the extremities; decreased or absent pulses; high blood pressure; headaches; visual disturbances;
- thromboangiitis obliterans: pain in the hands, arms, feet and legs; ulcers on the fingers and toes;
- Wegener's disease: nasal stuffiness; chronic sinus infections; nosebleeds.

**"Therapeutically effective amount"** means level or amount of agent that is aimed at, without causing significant negative or adverse side effects to the target, (1) delaying or preventing the onset of a vasculitis; (2) slowing down or stopping the progression, aggravation, or deterioration of one or more symptoms of the vasculitis; (3) bringing about ameliorations of the symptoms of the vasculitis; (4) reducing the severity or incidence of the vasculitis; or (5) curing the vasculitis. A therapeutically effective amount may be administered prior to the onset of the vasculitis, for a prophylactic or preventive action. Alternatively or additionally, the therapeutically effective amount may be administered after initiation of the vasculitis, for a therapeutic action.

**"Treatment"** and any declensions thereof, with reference to a vasculitis, refer to the complete alleviation, inhibition, onset delay, or curation of the vasculitis and/or of symptoms or complications associated therewith.

### DETAILED DESCRIPTION

The present invention relates to anethole trithione (ATT), for use in preventing, treating, and/or lessening the severity or progression of, a vasculitis in a subject in need thereof. It also relates to a method for preventing, treating, and/or lessening the severity or progression of, a vasculitis in a subject in need thereof, comprising administering to said subject anethole trithione (ATT). It also relates to the use of anethole trithione (ATT) for the manufacture of a medicament for preventing, treating, and/or lessening the severity or progression of, a vasculitis in a subject in need thereof.

In the context of the invention, the type of vasculitis to be prevented or treated may be any kind of vasculitis, such as, without limitation:
- a large vessel vasculitis (including, but not limited to, giant cell arteritis, Takayasu arteritis);
- a medium vessel vasculitis (including, but not limited to, giant cell arteritis, Takayasu arteritis, polyarteritis nodosa, Kawasaki disease);
- a small vessel vasculitis (including, but not limited to, antineutrophil cytoplasmic antibody (ANCA)-associated vasculitis, microscopic polyangiitis, Wegener's disease, Churg-Strauss syndrome, anti-glomerular basement membrane (GBM) disease, Henoch-Schönlein purpura, cryoglobulinemic vasculitis, hypocomplementemic urticarial vasculitis, thromboangiitis obliterans);
- a variable vessel vasculitis (including, but not limited to, Behçet's disease, Cogan's disease);
- a single organ vasculitis (including, but not limited to, cutaneous leukocytoclastic angiitis, cutaneous arteritis, primary central nervous system vasculitis, isolated aortitis);
- a vasculitis associated with systemic disease (including, but not limited to, lupus vasculitis, rheumatoid vasculitis, sarcoid vasculitis); or
- a vasculitis associated with a probable etiology (including, but not limited to, hepatitis C virus-associated cryoglobulinemic vasculitis, hepatitis B virus-associated vasculitis, syphilis-associated aortitis, drug-associated immune complex vasculitis, drug-associated ANCA-associated vasculitis, cancer-associated vasculitis).

In one embodiment, the vasculitis is selected from the group comprising or consisting of large vessel vasculitides, medium vessel vasculitides, small vessel vasculitides, variable vessel vasculitides, single organ vasculitides, vasculitides associated with systemic disease and vasculitides associated with a probable etiology.

In one embodiment, the vasculitis is selected from the group comprising or consisting of Kawasaki disease, giant cell arteritis, Takayasu arteritis, polyarteritis nodosa, ANCA-associated vasculitis, microscopic polyangiitis, Wegener's disease, Churg-Strauss syndrome, anti-glomerular basement membrane (GBM) disease, Henoch-Schönlein purpura, cryoglobulinemic vasculitis, hypocomplementemic urticarial vasculitis, thromboangiitis obliterans, Behçet's disease, Cogan's disease, cutaneous leukocytoclastic angiitis, cutaneous arteritis, primary central nervous system vasculitis, isolated aortitis, lupus vasculitis, rheumatoid vasculitis, sarcoid vasculitis, hepatitis C virus-associated cryoglobulinemic vasculitis, hepatitis B virus-associated vasculitis, syphilis-associated aortitis, drug-associated immune complex vasculitis, drug-associated ANCA-associated vasculitis, and cancer-associated vasculitis.

In one embodiment, the vasculitis is selected from the group comprising or consisting of Kawasaki disease, Takayasu arteritis, polyarteritis nodosa, ANCA-associated vasculitis, microscopic polyangiitis, Churg-Strauss syndrome, anti-glomerular basement membrane (GBM) disease, Henoch-Schönlein purpura, cryoglobulinemic vasculitis, hypocomplementemic urticarial vasculitis, thromboangiitis obliterans, Cogan's disease, cutaneous leukocytoclastic angiitis, cutaneous arteritis, primary central nervous system vasculitis, isolated aortitis, lupus vasculitis, rheumatoid vasculitis, sarcoid vasculitis, hepatitis C virus-associated cryoglobulinemic vasculitis, hepatitis B virus-associated vasculitis, syphilis-associated aortitis, drug-associated immune complex vasculitis, drug-associated ANCA-associated vasculitis, and cancer-associated vasculitis.

In one embodiment, the vasculitis is selected from the group comprising or consisting of Kawasaki disease, ANCA-associated vasculitis, giant cell arteritis, Takayasu's arteritis and Behcet's disease.

In one embodiment, the vasculitis is selected from the group comprising or consisting of Kawasaki disease, ANCA-associated vasculitis and Takayasu's arteritis.

In one embodiment, the vasculitis is a granulomatous vasculitis.

In one embodiment, the granulomatous vasculitis is selected from the group comprising or consisting of Kawasaki disease, giant cell arteritis, Takayasu arteritis, ANCA-associated vasculitis, Wegener's disease, Churg-Strauss syndrome, and primary central nervous system vasculitis.

In one embodiment, the granulomatous vasculitis is selected from the group comprising or consisting of Kawasaki disease, Takayasu arteritis, ANCA-associated vasculitis, Churg-Strauss syndrome, and primary central nervous system vasculitis.

In one embodiment, the vasculitis is Kawasaki disease.

In one embodiment, the vasculitis is Kawa-COVID-19. Kawa-COVID-19 (also named "multisystem inflammatory syndrome in children" [MIS-C], "multisystem inflammatory syndrome [MIS] in children and adolescents temporally related to COVID-19", "paediatric inflammatory multisystem syndrome [PIMS] temporally associated with SARS-CoV-2 infection" [PIMS-TS] or "systemic inflammatory syndrome in COVID-19" [SISCoV]) is a Kawasaki-like disease affecting young subjects (typically children and teenagers, and especially children under the age of 5) infected with the SARS-CoV-2 virus responsible for the 2019-2021 Covid-19 pandemic (Pouletty et al., 2020. Ann Rheum Dis. 79(8):999-1006; Dhar et al., 2021. Pediatr Res. Online ahead of print).

In the context of the invention, the term anethole trithione refers to 5-(4-methoxyphenyl)-3*H*-1,2-dithiole-3-thione, but is also meant to encompass pharmaceutically acceptable salts or solvates thereof, as well as structural analogs such as those of formula (I), (II) or (III) described in WO2018162581, which compounds are herein incorporated by reference.

In one embodiment, a structural analog of anethole trithione is a compound of formula (I): 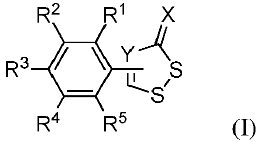 or a pharmaceutically acceptable tautomer, salt or solvate thereof wherein:
**X** represents S, O or NHOH; preferably **X** is S or O; more preferably, **X** is S;
**Y** represents CH, C or N; preferably **Y** is CH or N; more preferably **Y** is CH;
**R¹**, **R²**, **R⁴** and **R⁵** each independently represent hydrogen, hydroxy, halo, amino, alkylsulfonyl, aminosulfonyl, cyano, nitro, carboxy, aryl, alkoxy, haloalkyl, alkylamino, aminoalkyl, nitrooxyalkyl or carboxyalkyl;
**R³** is methoxy or hydroxy; or **R³** and **R²** together with the carbon atoms to which they are attached form a 5-membered heteroaryl moiety wherein **-R³-R²-** represents **-A-CR⁶=B-** or **-B=CR⁶-A-**; wherein:
   **A** represents O, S or NR⁷; wherein **R⁷** represents hydrogen, C₁-C₈ alkyl or alkyloxycarbonyl;
   **B** represents CH or N; and
   **R⁶** represents hydrogen, hydroxy, halo, amino, alkylsulfonyl, aminosulfonyl, cyano, nitro, carboxy, aryl, alkoxy, haloalkyl, alkylamino, aminoalkyl, nitrooxyalkyl or carboxyalkyl.

In one embodiment, a structural analog of anethole trithione is a compound selected from the group comprising or consisting 5-(4-hydroxyphenyl)-3*H*-1,2-dithiole-3-thione; 5-(4-hydroxyphenyl)-3*H*-1,2-dithiol-3-one; 5-(4-hydroxyphenyl)-3*H*-1,2-dithiol-3-one oxime; 5-(4-hydroxyphenyl)-3*H*-1,2,4-dithiazole-3-thione; 4-(4-hydroxyphenyl)-3*H*-1,2-dithiole-3-thione; 5-(2-hydroxybenzo[*d*]oxazol-5-yl)-3*H*-1,2-dithiole-3-thione; 5-(2-hydroxybenzo[*d*]thiazol-6-yl)-3*H*-1,2-dithiole-3-thione; 5-(benzofuran-5-yl)-3*H*-1,2-dithiole-3-thione; and methyl 5-(3-thioxo-3*H*-1,2-dithiol-5-yl)-1*H*-indole-1-carboxylate.

In one embodiment, a structural analog of anethole trithione is 5-(4-hydroxyphenyl)-3*H*-1,2-dithiole-3-thione.

The present invention also encompasses a method for preventing, treating, and/or lessening the severity or progression of, a vasculitis in a subject in need thereof, comprising administering to said subject a composition comprising anethole trithione (ATT); and to uses of such composition comprising anethole trithione (ATT) for preventing, treating, and/or lessening the severity or progression of, a vasculitis in a subject in need thereof.

In one embodiment, the composition comprising anethole trithione (ATT) is a pharmaceutical composition, and further comprises a pharmaceutically acceptable excipient.

In one embodiment, anethole trithione is formulated for administration to the subject in need thereof.

In one embodiment, anethole trithione or the composition comprising the same is to be administered systemically or locally.

In one embodiment, anethole trithione or the composition comprising the same is to be administered by injection, orally, topically, nasally, buccally, rectally, vaginaly, intratracheally, by endoscopy, transmucosally, or by percutaneous administration.

In one embodiment, anethole trithione or the composition comprising the same is to be injected, preferably systemically injected.

Examples of systemic injections include, but are not limited to, intravenous (iv), subcutaneous (sq), intradermal (id), intramuscular (im), intraarterial, intraparenteral, intranodal, intralymphatic, intraperitoneal (ip), intracranial, intracardiac, intralesional, intraprostatic, intravaginal, intrarectal, intrathecal, intranasal, intratumoral (it), intravesicular, and perfusion.

Examples of formulations of anethole trithione adapted for systemic injections include those comprising sulfobutyl ether-beta-cyclodextrin described in WO2020049166, which formulations are herein incorporated by reference.

In one embodiment, anethole trithione or the composition comprising the same is to be administered orally.

Examples of formulations of anethole trithione adapted for oral administration include Felviten, Halpen, Hepasulfol, Heporal, Mucinol (Sanofi-Aventis), Sialor (Paladin Laboratories, Pharmascience, Solvay, Zuoz Pharma), Sonicur (Solvay), Sulfarlem (Solvay, Aguettant, Edward Keller, Sanofi-Aventis), Sulfarlem S (EG Labo), Tiopropen and Tiotrifar.

In one embodiment, anethole trithione or the composition comprising the same may be used in conjunction with delivery systems that facilitate delivery of anethole trithione to the central nervous system. For example, various blood brain barrier (BBB) permeability enhancers may be used to transiently and reversibly increase the permeability of the blood brain barrier to a drug. Such BBB permeability enhancers include, but are not limited to, leukotrienes, bradykinin agonists, histamine, tight junction disruptors *(e.g.,* zonulin, zot), hyperosmotic solutions *(e.g.,* mannitol), cytoskeletal contracting agents, and short chain alkylglycerols (*e.g.*, 1-*O*-pentylglycerol). Oral, sublingual, parenteral, implantation, nasal and inhalational routes can provide delivery of anethole trithione to the central nervous system. In some embodiments, anethole trithione or the composition comprising the same may be administered to the central nervous system of the subject with minimal effects on the peripheral nervous system.

It will be understood that other suitable routes of administration are also contemplated in the present invention, and the administration mode will ultimately be decided by the attending physician within the scope of sound medical judgment. Apart from administration by injection (iv, ip, im and the like), other routes are available, such as nebulization or subcutaneous administration.

It should also be understood that the dose administered to the subject will ultimately be decided by the attending physician and personally adapted to each subject within the scope of sound medical judgment. The specific therapeutically effective amount for any particular subject will depend upon a variety of factors including the specific condition being treated and the severity of the condition; the specific composition employed; the age, body weight, general health, sex and diet of the subject; the time of administration, route of administration, the duration of the treatment; drugs used in combination or coincidental with anethole trithione or the composition comprising the same; and like factors well known in the medical arts.

In one embodiment, anethole trithione is to be administered before, concomitantly with or after an additional therapeutic agent.

In one embodiment, anethole trithione is to be administered as an add-on therapy after the additional therapeutic agent.

As used herein, the term **"add-on therapy"** refers to a treatment given to bolster or enhance the therapeutic efficacy of a previous one (the additional therapeutic agent), and/or to alleviate the side effects associated with the previous one (the additional therapeutic agent).

Suitable additional therapeutic agents include, but are not limited to, intravenous immunoglobulin (IVIg), aspirin, corticosteroids, immunosuppressor agents and alkylating agents.

As used herein, **"intravenous immunoglobulin"** or **"IVIg"** refers to an immunoglobulin therapy using a mixture of "normal human immunoglobulin" (NHIG). IVIg are typically prepared from pools of at least 1000 donations of human plasma; it contains immunoglobulin G (IgG) and antibodies to a number of viruses that are prevalent in the general population. IVIg is commercially available under several brand names, such as Aragam^{®} (Oxbridge), Flebogamma^{®} DIF (Grifols), Gammagard S/D^{®} (Baxter), Gammaplex^{®} (BPL), Gamunex^{®} (Grifols), Intratect^{®} (Biotest UK), Kiovig^{®} (Baxter), Octagam^{®} (Octapharma), Privigen^{®} (CSL Behring), and Vigam^{®} (BPL).

Suitable examples of corticosteroids include those described under subgroup H02 of the Anatomical Therapeutic Chemical Classification System.

Further suitable examples of corticosteroids include, but are not limited to:
(i) glucocorticoids, such as, *e.g.:*
   a. natural glucocorticoids, including cortisone, cortodoxone, desoxycortone, hydrocortisone, prebediolone, pregnenolone, and the like;
   b. synthetic glucocorticoids, including chloroprednisone, cloprednol, difluprednate, fludrocortisone, flugestone acetate, fluocinolone, fluorometholone, fluperolone, fluprednisolone, loteprednol, medrysone, methylprednisolone, prednicarbate, prednisolone, prednisone, tixocortol, alclometasone, beclomethasone, betamethasone, clobetasol, clobetasone, clocortolone, cortivazol, desoximetasone, dexamethasone, diflorasone, diflucortolone, fluclorolone, flumetasone, fluocortin, fluocortolone, fluprednidene, fluticasone, halometasone, meprednisone, mometasone, paramethasone, prednylidene, rimexolone, triamcinolone, ulobetasol, amcinonide, budesonide, ciclesonide, deflazacort, desonide, flucloronide, fludroxycortide, flunisolide, fluocinolone acetonide, fluocinonide, formocortal, halcinonide, triamcinolone acetonide, and the like;
(ii) mineralocorticoids, such as, *e.g*.: desoxycortone, hydrocortisone, fludrocortisone, methylprednisolone, prednisolone, prednisone, and the like.

Suitable examples of immunosuppressor agents include those described under subgroup L04 of the Anatomical Therapeutic Chemical Classification System.

Further suitable examples of immunosuppressor agents include, but are not limited to:
(i) antimetabolites, such as, *e.g.:*
   a. antifolates, including aminopterin, methotrexate, pemetrexed, pralatrexate, pteropterin, raltitrexed, denopterin, trimetrexate, pemetrexed, and the like;
   b. purine analogues, including azathioprine, mycophenolic acid, mycophenolate mofetil, pentostatin, cladribine, clofarabine, fludarabine, nelarabine, tioguanine, mercaptopurine, and the like;
   c. pyrimidine analogues, including leflunomide, teriflunomide, fluorouracil, capecitabine, doxifluridine, tegafur, tegafur/gimeracil/oteracil, carmofur, floxuridine, cytarabine, gemcitabine, azacytidine, decitabine, and the like; and
   d. hydroxycarbamide;
(ii) macrolides, such as, *e.g*., tacrolimus, ciclosporin, pimecrolimus, abetimus, gusperimus, and the like;
(iii) immunomodulatory imide drugs, such as, *e.g*., lenalidomide, pomalidomide, thalidomide, apremilast, and the like;
(iv) IL-1 receptor antagonists, such as, *e.g.,* anakinra, and the like);
(v) mTOR inhibitors, such as, *e.g*., sirolimus, everolimus, ridaforolimus, temsirolimus, umirolimus, zotarolimus, and the like);
(vi) serum-targeting antibodies, such as, *e.g*., anti-complement component 5 antibodies (*e.g.,* eculizumab), anti-TNF antibodies (*e.g.,* adalimumab, afelimomab, certolizumab pegol, etanercept, golimumab, infliximab, nerelimomab, pegsunercept), anti-interleukin 5 antibodies (*e.g.,* mepolizumab), anti-immunoglobulin E antibodies (*e.g.,* omalizumab), anti-interferon antibodies (*e.g.,* faralimomab), anti-interleukin 6 antibodies (*e.g*., elsilimomab, filgotinib), anti-interleukin 12/interleukin 23 antibodies (*e.g*., lebrikizumab, ustekinumab), anti-interleukin 17A antibodies (*e.g*., secukinumab), and the like;
(vii) cell-targeting antibodies, such as, *e.g.,* anti-CD3 antibodies *(e.g.,* muromonab-CD3, otelixizumab, teplizumab, visilizumab), anti-CD4 antibodies (*e.g*., clenoliximab, keliximab, zanolimumab), anti-CDlla antibodies (*e.g*., efalizumab), anti-CD18 antibodies (*e.g.,* erlizumab), anti-CD20 antibodies *(e.g.,* obinutuzumab, rituximab, ocrelizumab, pascolizumab), anti-CD23 antibodies (*e.g*., gomiliximab, lumiliximab), anti-CD40 antibodies (*e.g*., teneliximab, toralizumab), anti-CD62L antibodies *(e.g.,* aselizumab), anti-CD80 antibodies *(e.g.,* galiximab), anti-CD147 antibodies *(e.g.,* gavilimomab), anti-CD154 antibodies *(e.g.,* ruplizumab), anti-BLysS antibodies *(e.g.,* belimumab, blisibimod), anti-CTLA-4 antibodies (*e.g.,* abatacept, belatacept), anti-interleukin 2 receptor antibodies (*e.g*., basiliximab, daclizumab, inolimomab), anti-interleukin 6 receptor antibodies (*e.g.,* tocilizumab), anti-integrin antibodies (*e.g*., natalizumab, vedolizumab), and the like.

Suitable examples of alkylating agents include, but are not limited to, nitrogen mustards (such as, *e.g*., chlormethine, cyclophosphamide, ifosfamide, trofosfamide, chlorambucil, melphalan, prednimustine, bendamustine, uramustine, and the like), nitrosoureas (such as, *e.g*., carmustine, lomustine, semustine, fotemustine, nimustine, ranimustine, streptozocin, and the like), alkyl sulfonates (such as, *e.g.,* busulfan, mannosulfan, treosulfan, and the like), aziridines (such as, *e.g.,* carboquone, thiotepa, triaziquone, triethylenemelamine, benzodopa, meturedopa, uredopa, and the like), hydrazines (such as, *e.g.,* procarbazine, and the like), triazenes (such as, *e.g.,* dacarbazine, temozolomide, and the like), altretamine, mitobronitol, pipobroman, actinomycin, bleomycin, mitomycins and plicamycin.

In one embodiment, anethole trithione is to be administered before, concomitantly with or after intravenous immunoglobulin (IVIg).

In one embodiment, the subject is an animal, preferably a mammal, more preferably a primate, even more preferably a human.

In one embodiment, the subject is a male. In one embodiment, the subject is a female.

In one embodiment, the subject is a child, *i.e.,* a young human being below the age of puberty or alternatively below the legal age of majority. In one embodiment, the child is a newborn, *i.e.,* a young human in the first 28 days after birth. In one embodiment, the child is an infant, *i.e.,* a young human between 1 and 12 months old. In one embodiment, the child is a toddler, *i.e.,* a young human between 12 and 36 months old. In one embodiment, the subject is a teenager, *i.e.,* a young human being between the age of puberty and the legal age of majority, such as 18, 19, 20 or 21 years old.

In one embodiment, the subject is a child under 3, 4, 5, 6, 7, 8, 9, 10, 11, or 12 months of age. In one embodiment, the subject is a child under 2, 3, 4, 5, 6, 7, 8, 9, or 10 years of age.

In one embodiment, the subject is a child between 3 and 12 months of age. In one embodiment, the subject is a child under 5 years of age.

In one embodiment, the subject is an adult, *i.e.,* a human being above the legal age of majority (such as above 18, 19, 20 or 21 years old) or alternatively above the age of puberty.

In one embodiment, the subject is/was diagnosed with a vasculitis. In one embodiment, the subject is/was diagnosed with Kawasaki disease. In one embodiment, the subject is/was diagnosed with Kawa-COVID-19.

The present invention also relates to a method for reducing the contractility of arterial vessels with endothelial dysfunction and preserving arterial vessel relaxation in a subject in need thereof, comprising administering to said subject anethole trithione (ATT) or a composition comprising the same; and to uses of anethole trithione (ATT) or of a composition comprising the same for reducing the contractility of arterial vessels with endothelial dysfunction and preserving arterial vessel relaxation.

By **"preserving arterial vessel relaxation",** it is meant preventing arterial vessel vasoconstriction and/or promoting arterial vessel vasodilation.

In one embodiment, endothelial dysfunction is an arterial vessel de-endothelialization.

Hence, the present invention also finds its use in the prevention, treatment, and/or lessening of the severity or progression of diseases associated with or characterized by an hypercontractility of arterial vessels with endothelial dysfunction, in particular of de-endothelialized arterial vessels, and/or associated with or characterized by arterial vessel vasoconstriction.

Such diseases, also referred to as "vasoconstrictive disorders" or "vasospastic diseases", include, without limitation, coronary vasospasm, vasospastic angina, heart attack, diabetic neuropathy, diabetic gangrene, vascular headache, cerebral vasospasm, reversible cerebral vasoconstriction syndrome (RCVS), Raynaud's disease, acrocyanosis, Buerger's disease, complex regional pain syndrome (CRPS), livedo reticularis, seizures, and post-traumatic dystrophy.

The present invention also relates to a method for inhibiting the secretion of soluble factors by macrophages, in particular of TNF-α using anethole trithione (ATT), and to uses of anethole trithione (ATT) for inhibiting the secretion of soluble factors by macrophages, in particular of TNF-α.

Hence, the present invention also finds its use in the prevention, treatment, and/or lessening of the severity or progression of diseases associated with or characterized by the secretion of soluble factors by macrophages, in particular of TNF-α.

### BRIEF DESCRIPTION OF THE DRAWINGS

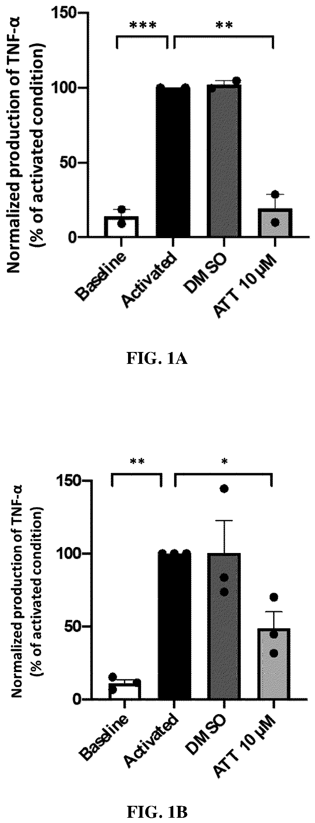

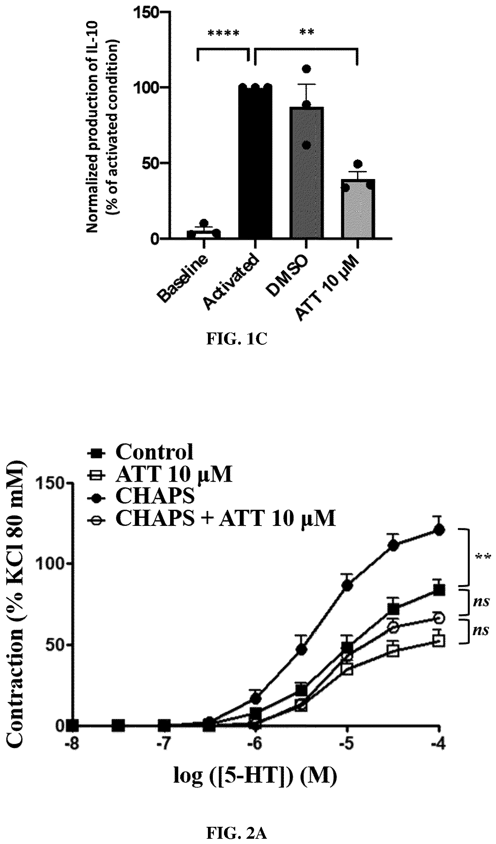

**Figures 1A-C** are a set of three graphs showing the effect of ATT on cytokine production by macrophages. **Fig. 1A** shows ATT inhibition of TNF-α production in LPS-activated macrophages. Dunnett's multiple comparisons test: ** *p* < 0.01; *** *p* < 0.001. **Fig. 1B** shows the reduced production of TNF-α by LPS-activated human macrophages cultured with ATT. Dunnett's multiple comparisons test: * *p* < 0.05; ** *p* < 0.01. **Fig. 1C** shows the reduced production of IL-10 by LPS-activated human macrophages cultured with ATT. Dunnett's multiple comparisons test: ** *p* < 0.01; **** *p* < 0.0001.

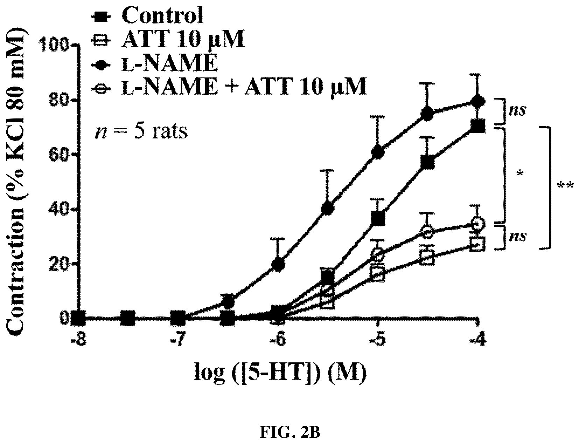

**Figures 2A-B** are a set of two graphs showing the effect of ATT on 5-HT reactivity of pulmonary arterial rings in the presence of endothelium dysfunction mediated by CHAPS or L-NAME. **Fig. 2A** shows the 5-HT reactivity in the presence of endothelium dysfunction mediated by CHAPS. Dunnett's multiple comparisons test: *** p* < 0.01; *ns:* not significant. **Fig. 2B** shows the 5-HT reactivity in the presence of endothelium dysfunction mediated by L-NAME. Dunnett's multiple comparisons test: * *p* < 0.05; ** *p* < 0.01; *ns:* not significant.

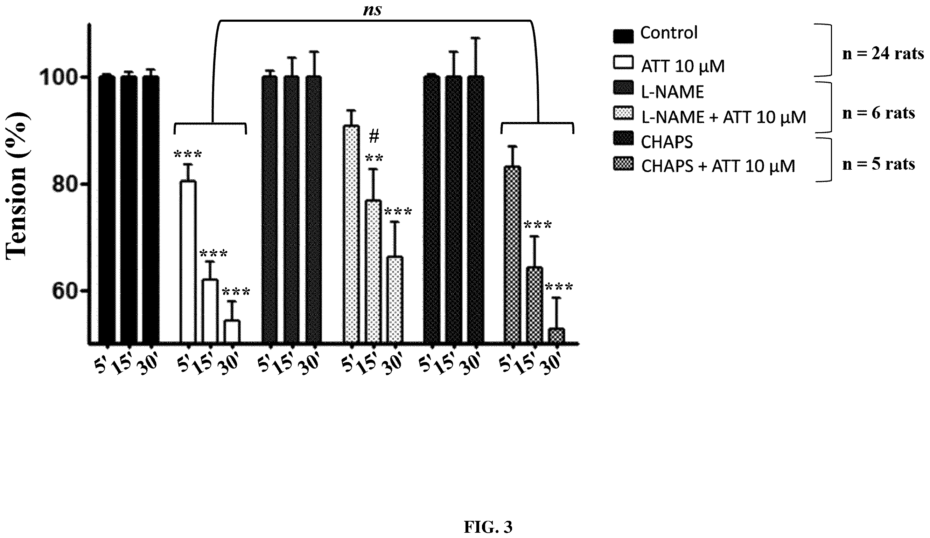

**Figure 3** is a graph showing the relaxing effect of ATT on phenylephrine-induced contraction of pulmonary arterial rings in the presence of endothelium dysfunction mediated by CHAPS or L-NAME. The tension percentages are normalized with respect to the control tension percentages for each group (ATT *vs.* Control; L-NAME+ATT *vs*. L-NAME; CHAPS+ATT *vs*. CHAPS). Dunnett's multiple comparisons test: * *p* < 0.05; ** *p* < 0.01; *ns:* not significant.

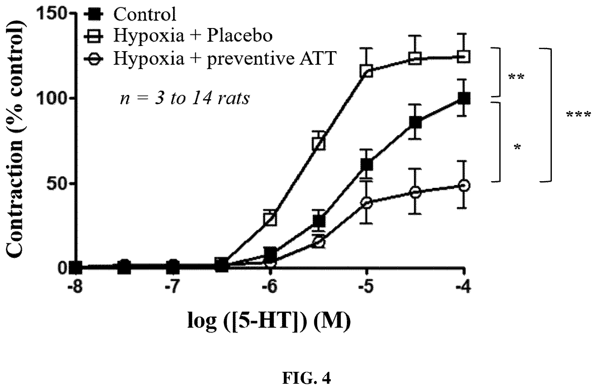

**Figure 4** is a graph showing the effect of acute or preventive treatment with ATT on 5-HT reactivity of pulmonary arterial rings from rats with PH. Data are expressed in percentage of control contraction. Statistical analysis: two-way ANOVA and Bonferroni post-test. * *p* < 0.05; ** *p* < 0.01; *** *p* < 0.001; *ns:* not significant.

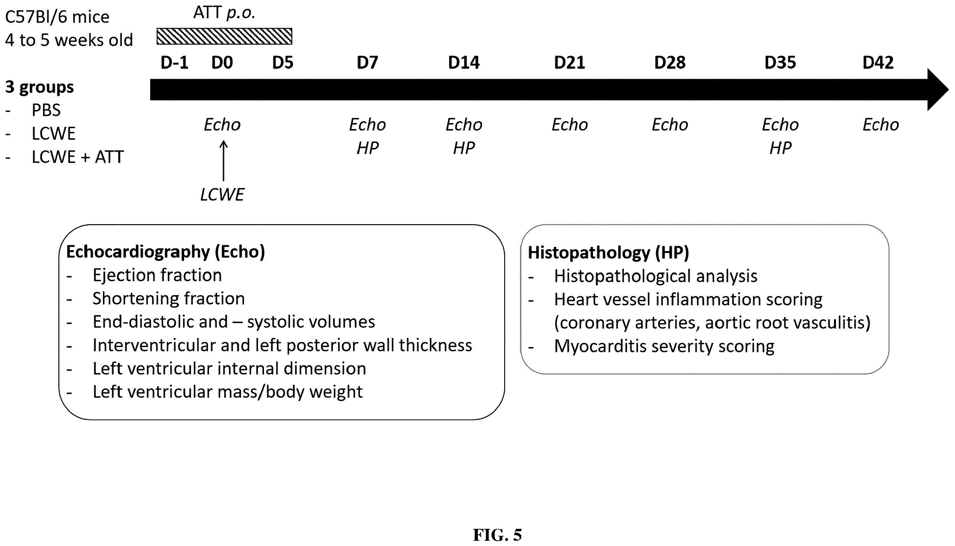

**Figure 5** is a scheme of the *in vivo* protocol used to evaluate the effect of ATT in a LCWE-induced Kawasaki disease mouse model.

### EXAMPLES

The present invention is further illustrated by the following examples.

### Example 1

### Evaluation of ATT on cytokine production by macrophages

### Materials and Methods

CD14⁺ human macrophages isolated from healthy donors were cultured with LPS (1 µg/mL) and ATT (10 µM) for 48 hours. At the end of the culture, the supernatants were collected and cytokine production was evaluated.

Production of TNF-α was measured by ELISA. Production of TNF-α and IL-10 were measured using a multiplex detection technique.

### Results

Vasculitis is characterized by macrophages and lymphocytes activation resulting in inflammatory cytokines release. Since macrophages activation results in inflammatory cytokines release, the aim of the study was to assess the effects of ATT (10 µM) on its ability to inhibit the production of these cytokines.

As illustrated in **Fig. 1A****,** ATT significantly reduced the production of TNF-α in LPS-activated human macrophages (-88% *vs*. LPS-activated condition; Dunnett's multiple comparisons test: *p* = 0.001).

The multiplex assay confirmed the ELISA results, namely that TNF-α level decreased in LPS-activated human macrophages in presence of ATT (- 51.2 %) (**Fig. 1B**). Additionally, ATT significantly decreased the production of IL-10 (- 60.3 %) (**Fig. 1C**).

### Conclusion

TNF-α is a pleiotropic molecule known to be involved in a number of pathological conditions including vasculitis such as Kawasaki disease (Shen et al., 2013. Biochem Biophys Res Commun. 437(2):250-5).

The present results demonstrate that ATT is capable of reducing the cytokine release of activated human macrophages, clearly suggesting a possible therapeutic effect of ATT against vasculitides, and in particular, Kawasaki disease.

### Example 2

### Evaluation of ATT on vessel reactivity in presence of endothelium dysfunction

### Materials and Methods

Intrapulmonary arteries of the first order (IPA1) of Wistar rats (male, 300 g) were divided into short tubular segments with an external diameter of ≈ 1.5-2 mm and used for isometric contraction measurement.

Arterial rings were mounted in isolated organ bath systems (EMKA Technologies), containing Krebs solution (118 mM NaCl, 4.7 mM KCl, 1.2 mM MgSO₄, 2.5 mM NaHCO₃, 1.2 mM KH₂PO₄, 2.5 mM CaCl₂, and 11 mM D-glucose, pH 7.4) at 37°C and bubbled continuously with 15 % O₂/5 % CO₂.

Arterial rings were stretched to a basal tension of 0.8 to 1.0 g according to their diameter. Tissues were allowed to equilibrate for 1 hour in Krebs solution and washed out every 15 minutes. A high KCl solution (80 mM) was applied in order to obtain a reference contraction used to normalize subsequent contractile responses.

At the end of the experiment, endothelial function was tested on each ring by examining the relaxation induced by carbamylcholine, a potent vascular relaxing agent (100 µM; Sigma) on 1 µM or 30 nM Phe-induced pre-constricted pulmonary arterial rings. Passive and active mechanical properties were assessed using transducer systems, coupled to IOX2 software (v2.10.8, EMKA technologies, Paris, France) in order to facilitate data acquisition and analysis. Data were then analyzed using two-way ANOVA with Bonferroni post-tests (Prism 5 v.5.01).

### Contractile function

Contractile responses were tested by constructing a cumulative concentration-response curve (CCRC) to 5-hydroxytryptamine [5-HT] (10 nM to 100 µM; Sigma). When indicated, tissues were preincubated during 30 minutes with ATT (10 µM), and then CCRC to 5-HT was performed in the presence or absence of the drug.

Evaluation of the role of endothelium on ATT effect was assessed using N^{ω}-nitro-L-arginine methyl ester [L-NAME] (100 µM, 30 minutes preincubation; Sigma) to inhibit nitric oxide-dependent relaxation, or 3-[(3-cholamidopropyl)dimethylammonio]-1-propanesulfonate [CHAPS] (0.3 % in water, perfusion before mounting; Sigma) to physically damage the endothelium.

### Relaxation of the vessel

Relaxation was assessed by injecting ATT (10 µM) on phenylephrine [Phe] (30 nM; Sigma) pre-constricted pulmonary arterial rings when contraction reached its steady-state.

The role of endothelium on ATT relaxant effect was assessed as aforementioned with L-NAME pre-incubation or CHAPS perfusion. Endothelial function was tested on each ring by examining the relaxation induced by 100 µM carbamylcholine (Sigma) on 1 µM or 30 nM Phe-induced pre-constricted pulmonary arterial rings. Passive and active mechanical properties were assessed using transducer systems, coupled to IOX2 software (v2.10.8, EMKA technologies, Paris, France) in order to facilitate data acquisition and analysis. Data were then analyzed using two-way ANOVA with Bonferroni post-tests (Prism 5 v.5.01).

### Chronic endothelium dysfunction

In order to investigate the protective effects of ATT on chronic endothelium damage, rats were housed during 3 weeks in hypobaric hypoxia chambers (50 kPa or 10 % O₂).

Continuous treatment was induced through a minipump placed in the abdominal cavity to deliver ATT (0.7 mg/mL) or its placebo (sulfobutylether beta-cyclodextrin) during the whole hypoxia.

After euthanasia of the animal, pulmonary arterial rings were placed in an organ bath and the reactivity of the vessel was investigated by using cumulative concentration-response curve with 5-HT (10 nM to 100 µM). This *ex vivo* part of the study was conducted without any addition of ATT in the organ bath, meaning that the effect was exclusively related to the chronic *in vivo* treatment through the minipump.

### Results

### Protective effect of ATT on contractile function in the presence of endothelium dysfunction

As shown on **Fig. 2A****,** the concentration response curve in presence of CHAPS-induced endothelium dysfunction was shifted toward the left, meaning that the rings were more sensitive to 5-HT activation. Furthermore, the maximum of 5-HT efficacy was significantly potentiated by CHAPS incubation.

Surprisingly, the concentration response curve in presence of ATT and CHAPS and the maximum efficacy were not significantly different to that obtained in the presence of normal endothelium function. Therefore, the present result demonstrates that ATT could prevent endothelium damages induced by CHAPS, and could restore the normal contractility of the ring.

As shown on **Fig. 2B****,** the concentration response curve in the presence of L-NAME-induced endothelium dysfunction was also shifted toward the left, meaning that the rings were more sensitive to 5-HT activation.

In the presence of ATT, the concentration response curve with L-NAME was markedly shifted and the maximum effect was significantly reduced. Therefore, the present result demonstrates that ATT could prevent the alteration of nitric oxide-relaxing pathway induced by L-NAME, and could reduce the hypercontractility of the ring induced by 5-HT.

### Protective effect of ATT on the relaxation of the vessel in the presence of endothelium dysfunction

The amplitude of the relaxations to ATT alone or in the presence of L-NAME or CHAPS were expressed as a percentage of the maximal contraction to 30 nM phenylephrine normalized to 100 % in control conditions or in the presence of L-NAME or CHAPS respectively. As shown on **Fig. 3****,** the amplitude of relaxation was similar in the group of rings treated with ATT or with CHAPS + ATT.

Surprisingly, a pre-treatment with L-NAME or CHAPS to induce endothelium dysfunction failed to affect the relaxation mediated by ATT (**Fig. 3****,** L-NAME + ATT and CHAPS + ATT, respectively). On the contrary, the relaxation of carbamylcholine in similar condition was fully abolished by endothelium dysfunctions induced by L-NAME or CHAPS (data not shown).

Collectively, these results demonstrate that ATT-induced vessel relaxation is not affected by endothelium dysfunctions induced by CHAPS or L-NAME.

### In vivo protective effect of ATT on contractile function in the presence of chronic endothelium dysfunction

As shown on **Fig. 4****,** a chronic endothelium dysfunction induced by hypoxia generated a hyper-reactivity of the vessel induced by 5-HT. The chronic treatment with ATT fully abolished this hyperactivity of the vessel induced by 5-HT, confirming the protective effect of ATT against endothelium dysfunction in an *in vivo* model.

### Conclusion

Vasculitic syndromes, including Kawasaki disease, are known to be associated with cardiovascular manifestations and complications, in particular an arterial vessel dysfunction.

The present results demonstrate that ATT is capable of reducing hypercontractility of arterial vessels with endothelial dysfunction and to preserve vessel relaxation, hence clearly suggesting a possible therapeutic effect of ATT against vasculitides, and in particular, Kawasaki disease.

### Example 3

### Evaluation of ATT on an in vivo mouse model of Kawasaki disease

*Lactobacillus casei* is a Gram-positive bacterium that colonizes the gastrointestinal and urogenital tracts of both human and animals. The cardiovascular lesions induced in mice by *Lactobacillus casei* cell wall extract (LCWE) are histologically similar to those observed in human vasculitic syndromes. LCWE-induced Kawasaki disease is characterized by infiltration of innate immune cells in the aortic root, development of necrotizing arteritis in the coronary artery followed by luminal obstruction that can lead to complete coronary artery stenosis.

LCWE mouse model therefore closely mimics the histopathological and immune pathological features of the cardiovascular lesions in KD, and has been used in the efficacy evaluation of drug candidates currently tested in clinical trials (Lau et al., 2009. Clin Exp Immunol. 157(2):300-9; Gorelik et al., 2019. Clin Exp Immunol. 198(1): 101-110).

**Fig. 5** illustrates the protocol conducted on LCWE mouse model to evaluate the effect of ATT on LCWE-induced KD.

## Claims

1. Anethole trithione (ATT), for use in preventing, treating, and/or lessening the severity or progression of, a vasculitis in a subject in need thereof.

2. ATT for use according to claim **1,** wherein preventing, treating, and/or lessening the severity or progression of the vasculitis comprises reducing or inhibiting macrophage activation.

3. ATT for use according to claim **2,** wherein reducing or inhibiting macrophage activation comprises reducing the release of tumor necrosis factor (TNF)-α by said macrophages.

4. ATT for use according to any one of claims **1** to **3,** wherein preventing, treating, and/or lessening the severity or progression of the vasculitis comprises reducing the contractility of arterial vessels with endothelial dysfunction and/or preventing arterial vessel vasoconstriction and/or promoting arterial vessel vasodilation.

5. ATT for use according to any one of claims **1** to **4,** wherein preventing, treating, and/or lessening the severity or progression of the vasculitis comprises reducing the contractility of de-endothelialized arterial vessels.

6. ATT for use according to any one of claims **1** to **5,** wherein the vasculitis is selected from the group consisting of Kawasaki disease, giant cell arteritis, Takayasu arteritis, polyarteritis nodosa, antineutrophil cytoplasmic antibody (ANCA)-associated vasculitis, microscopic polyangiitis, Wegener's disease, Churg-Strauss syndrome, anti-glomerular basement membrane (GBM) disease, Henoch-Schönlein purpura, cryoglobulinemic vasculitis, hypocomplementemic urticarial vasculitis, thromboangiitis obliterans, Behçet's disease, Cogan's disease, cutaneous leukocytoclastic angiitis, cutaneous arteritis, primary central nervous system vasculitis, isolated aortitis, lupus vasculitis, rheumatoid vasculitis, sarcoid vasculitis, hepatitis C virus-associated cryoglobulinemic vasculitis, hepatitis B virus-associated vasculitis, syphilis-associated aortitis, drug-associated immune complex vasculitis, drug-associated ANCA-associated vasculitis, and cancer-associated vasculitis.

7. ATT for use according to any one of claims **1** to **6,** wherein the vasculitis is Kawasaki disease.

8. ATT for use according to any one of claims **1** to **7,** wherein the vasculitis is Kawa-COVID-19.

9. ATT for use according to any one of claims **1** to **8,** wherein ATT is to be administered before, concomitantly with or after an additional therapeutic agent.

10. ATT for use according to claim **9,** wherein the additional therapeutic agent is selected from the group consisting of intravenous immunoglobulin (IVIg), aspirin, corticosteroids, immunosuppressor agents and alkylating agents.

11. ATT for use according to claim **9** or **10,** wherein the additional therapeutic agent is IVIg.

12. ATT for use according to any one of claims **1** to **11,** wherein the subject is a child or a teenager.
